# EUROPEAN PATENT SPECIFICATION

(11) **EP 4 475 766 B1**
(45) Date of publication and mention of the grant of the patent: **30.09.2026**
(21) Application number: 23701359.4
(22) Date of filing: 19.01.2023
(51) Int. Cl.: G06T 7/13, A61B 8/08, G06T 7/00, G16H 50/30, A61B 8/00, G16H 30/40, G16H 50/20, G16H 50/70

(54) **AUTOMATED PLEURAL LINE ASSESSMENT IN LUNG ULTRASOUND**
AUTOMATISIERTE PLEURALE LINIENBEURTEILUNG IN LUNGENULTRASCHALL
ÉVALUATION AUTOMATISÉE DE LIGNE PLEURALE DANS DES ULTRASONS PULMONAIRES

(30) Priority: 10.02.2022 US 202263308625 P
(43) Date of publication of application: 18.12.2024
(73) Proprietor: Koninklijke Philips N.V., 5656 AE Eindhoven (NL)
(72) Inventor: CHEN, Alvin, 5656 AG Eindhoven (NL); RAJU, Balasundar Iyyavu, 5656 AG Eindhoven (NL); KRUECKER, Jochen, 5656 AG Eindhoven (NL)
(74) Representative: Philips Intellectual Property & Standards
(86) International application number: PCT/EP2023/051157
(87) International publication number: WO 2023/151911

(56) References cited:
- WO-A1-2022/016262
- CN-A- 111 297 396
- CN-A- 113 053 498
- IT-A1- 202000 009 667
- US-A1- 2020 359 991
- LA SALVIA MARCO ET AL: "Deep learning and lung ultrasound for Covid-19 pneumonia detection and severity classification", COMPUTERS IN BIOLOGY AND MEDICINE, NEW YORK, NY, US, vol. 136, 8 August 2021 (2021-08-08), XP086773414, ISSN: 0010-4825, [retrieved on 20210808], DOI: 10.1016/J.COMPBIOMED.2021.104742
- SOLDATI GINO ET AL: "‐19 : A Simple, Quantitative, Reproducible Method", JOURNAL OF ULTRASOUND IN MEDICINE., vol. 39, no. 7, 1 July 2020 (2020-07-01), United States, pages 1413 - 1419, XP055827857, ISSN: 0278-4297, Retrieved from the Internet <URL:http://aphp.aphp.fr/wp-content/blogs.dir/278/files/2020/09/2-Proposal-for-international-standardization-of-LUS-for-patients-with-Covid-19.-Soldati-J.-J-Ultrasound-Med-anglais-2020.pdf> [retrieved on 20230307], DOI: 10.1002/jum.15285
- ALMEIDA AITOR ET AL: "Lung ultrasound for point-of-care COVID-19 pneumonia stratification: computer-aided diagnostics in a smartphone. First experiences classifying semiology from public datasets", 2020 IEEE INTERNATIONAL ULTRASONICS SYMPOSIUM (IUS), IEEE, 7 September 2020 (2020-09-07), pages 1 - 4, XP033859598, DOI: 10.1109/IUS46767.2020.9251716

## Description

### BACKGROUND

In a lung, the pleural line indicates the interface between the soft tissues of the chest wall and the lung tissue. Recent clinical literature shows that changes in the pleural line, such as thickening or irregularity, are associated with the severity of respiratory and infectious diseases including COVID-19 pneumonia, as well as other lung pathologies. Other lung features such as B-lines and sub-pleural consolidations are directly linked to the position of the pleural line. Quantitative computer-based features, including pleural line features, have been shown to be individually suggestive of pathologies such as COVID-19. Evaluation of pleural line changes in lung ultrasound is subjective even for expert users, and under-trained users often lack confidence to make an assessment. Currently there is no objective method for providing an indication of pleural line changes to users, though such changes are seen in respiratory and infectious diseases such as pneumonia including COVID-19 pneumonia. Lung ultrasound has prognosis capabilities for longitudinal monitoring of a patient, but such a scheme requires an objective assessment of features such as pleural line changes, especially one that is automated, standardized, and explainable to the user.

Reference is made to the document LA SALVIA MARCO ET AL: "Deep learning and lung ultrasound for Covid-19 pneumonia detection and severity classification", COMPUTERS IN BIOLOGY AND MEDICINE, NEW YORK, NY, US, vol. 136, 8 August 2021 (2021-08-08). This discloses a method for training a deep learning model for Covid-19 pneumonia detection.

Reference is further made to the document SOLDATI GINO ET AL: Proposal for International Standardization for the use of lung ultrasound for patients with covid-19", JOURNAL OF ULTRASOUND IN MEDICINE., vol. 39, no. 7, 1 July 2020 (2020-07-01 ), pages 1413-1419. United States. This discloses a standard manual scoring system for lung ultrasound images.

Reference is further made to IT 2020 0000 9667 A1. This discloses a method for determining a stage of pneumonia from lung ultrasound images.

Documents US2020/359991A1, CN111297396A, CN113053498A and WO2022/016262A1 also disclose pleural line assessment in ultrasound images.

### SUMMARY

The invention is defined by the independent claims. Advantageous embodiments are provided in the dependent claims.

According to an aspect of the present disclosure that is not within the scope of the claims, a method for processing an ultrasound frame is provided. The method includes obtaining an ultrasound frame of a lung and identifying a pleural line in the lung; quantifying the pleural line identified in the lung to obtain a quantification of the pleural line; comparing the quantification of the pleural line to a predetermined value that characterizes a normal, healthy pleural line; and identifying at least one of irregularity or thickening in the pleural line based on comparing the quantification of the pleural line to the predetermined value.

According to another aspect of the present disclosure, a system for processing an ultrasound frame is provided in claim 11.

According to another aspect of the present disclosure, a tangible non-transitory computer readable storage medium that stores a computer program is provided in claim 1.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1A illustrates a system for automated pleural line assessment in lung ultrasound, in accordance with a representative embodiment.
FIG. 1B illustrates a device for automated pleural line assessment in lung ultrasound, in accordance with a representative embodiment.
FIG. 1C illustrates a controller for automated pleural line assessment in lung ultrasound, in accordance with a representative embodiment.
FIG. 2A illustrates a method for automated pleural line assessment in lung ultrasound, in accordance with a representative example.
FIG. 2B illustrates another method for automated pleural line assessment in lung ultrasound, in accordance with a representative example.
FIG. 2C illustrates another method for automated pleural line assessment in lung ultrasound, in accordance with a representative example.
FIG. 3A illustrates a visualization of a pleural line detected using an image processing algorithm, in accordance with a representative embodiment.
FIG. 3B illustrates a visualization of peak detection signal processing and a lung sliding motion map, in accordance with a representative embodiment.
FIG. 3C illustrates a visualization of a segmented pleural line, in accordance with a representative embodiment.
FIG. 3D illustrates detected forms of irregularity in the pleural line, in accordance with a representative embodiment.
FIG. 4A illustrates a visualization of an irregular pleural line, in accordance with a representative embodiment.
FIG. 4B illustrates a visualization of a thickened pleural line, in accordance with a representative embodiment.
FIG. 5A illustrates a dashboard for automated pleural line assessment in lung ultrasound, in accordance with a representative embodiment.
FIG. 5B illustrates output options for the dashboard shown in FIG. 5A, in accordance with a representative embodiment.
FIG. 6A illustrates an automatically-detected pleural line and corresponding quantifications, in accordance with a representative embodiment.
FIG. 6B illustrates another automatically-detected pleural line and corresponding quantifications, in accordance with a representative embodiment.
FIG. 6C illustrates another automatically-detected pleural line and corresponding quantifications, in accordance with a representative embodiment.
FIG. 6D illustrates another automatically-detected pleural line and corresponding quantifications, in accordance with a representative embodiment.
FIG. 7 illustrates a computer system, on which a method for automated pleural line assessment in lung ultrasound is implemented, in accordance with another representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation, and methods of manufacture may be omitted so as to avoid obscuring the description of the representative embodiments. Nonetheless, systems, devices, materials, and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component.

The terminology used herein is for purposes of describing particular embodiments only and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

As described herein, a lung ultrasound system may automatically detect the pleural line and display features indicative of changes of the pleural line including thickness, smoothness, and intensity variations along its contour. The ability to detect and display changes in the pleural line enables objective clinical decision-making for triage decisions, such as whether or not to admit a patient, and enhances the ability to accurately determine a prognosis after hospital admission by enabling longitudinal assessment over time. The lung ultrasound system described herein may be combined with other lung ultrasound features such as B-lines and consolidation. The lung ultrasound system may implement one or more automated, standardized, and explainable algorithm(s) for assessment of pleural line changes. The lung ultrasound system may also aggregate pleural line features for prediction of pleural abnormality, summary of pleural line changes, and display of the prediction and summary.

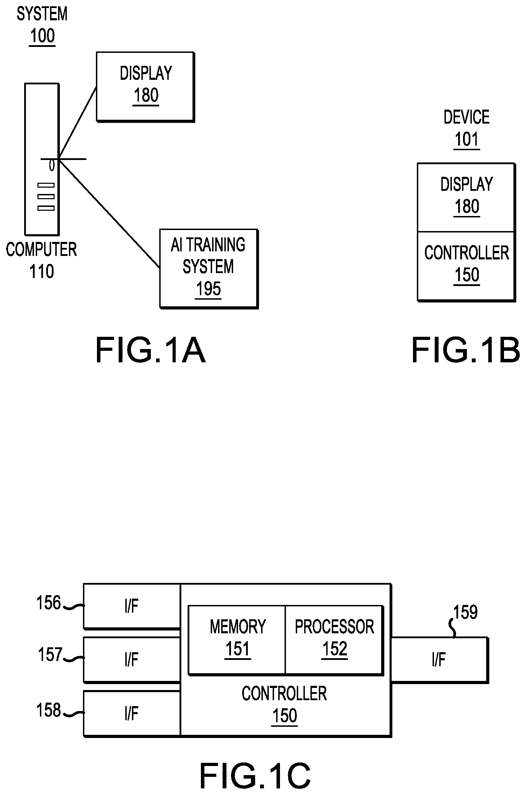

FIG. 1A illustrates a system for automated pleural line assessment in lung ultrasound, in accordance with a representative embodiment.

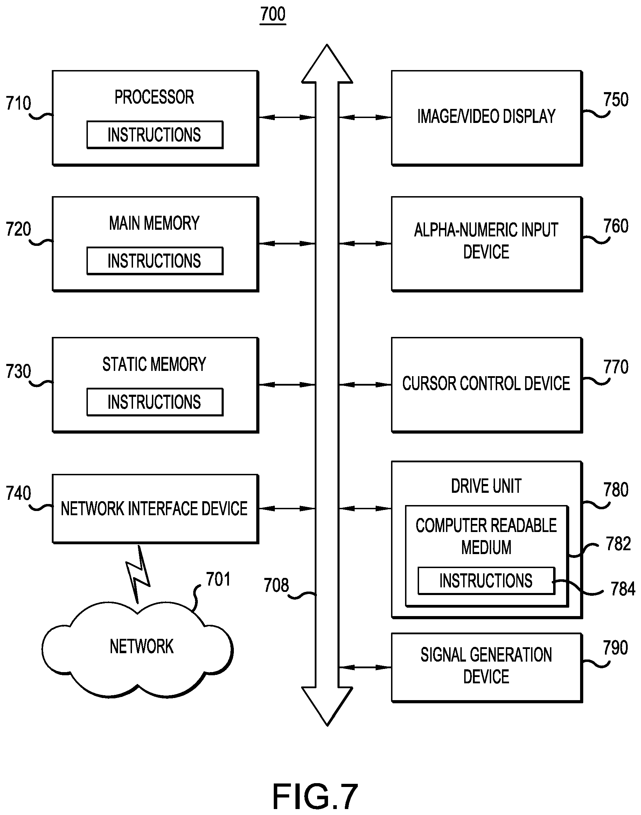

The system 100 includes a computer 110, a display 180 and an AI training system 195. The system 100 is a system for automated pleural line assessment in lung ultrasound and includes components that may be provided together or that may be distributed. The computer 110 includes a controller 150 depicted in FIG. 1C, and the controller 150 includes at least a memory 151 that stores instructions and a processor 152 that executes the instructions. The computer 110 may also include one or more input interface(s) that connect to other elements or components, such as the display 180. A computer that can be used to implement the computer 110 is depicted in FIG. 7, though a computer 110 may include more or fewer elements than depicted in FIG. 1A or in FIG. 7. The computer 110 may be interfaced with user input devices by which users can input instructions, including mouses, keyboards, thumbwheels and so on.

The display 180 may be local to the computer 110 or may be remotely connected to the computer 110. The display 180 may be connected to the computer 110 via a local wired interface such as an Ethernet cable or via a local wireless interface such as a Wi-Fi connection. The display 180 may be interfaced with user input devices by which users can input instructions, including mouses, keyboards, thumbwheels and so on.

The display 180 may be a monitor such as a computer monitor, a display on a mobile device, an augmented reality display, a television, an electronic whiteboard, or another screen configured to display electronic imagery. The display 180 may also include one or more input interface(s) that connect to other elements or components, such as the computer 110, as well as an interactive touch screen configured to display prompts to users and collect touch input from users.

The AI training system 195 includes computers used to train artificial intelligence. The AI training system 195 may be provided by the same entity that provides the computer 110 and the display 180, or a different entity. For example, a software development firm may use the AI training system 195 to train artificial intelligence models for sale to end users who use computers such as the computer 110 and displays such as the display 180. The AI training system 195 may develop and generate trained artificial intelligence. Instances of trained artificial intelligence that may be developed and generated by the AI training system 195 may include trained artificial intelligence that detects pleural lines in ultrasound frames. Instances of trained artificial intelligence that may be developed and generated by the AI training system 195 also may include trained artificial intelligence that detects pleural line features and predicts pleural abnormality based on quantification of the pleural line as described herein.

FIG. 1B illustrates a device for automated pleural line assessment in lung ultrasound, in accordance with a representative embodiment.

In FIG. 1B, the device 101 includes the controller 150 and the display 180. The device 101 illustrates that the automated pleural line assessment in lung ultrasound may be implemented using a single device, and does not necessarily require separate or distributed components such as the computer 110 and the display 180 in FIG. 1A.

FIG. 1C illustrates a controller for automated pleural line assessment in lung ultrasound, in accordance with a representative embodiment.

The controller 150 includes a memory 151, a processor 152, a first interface 156, a second interface 157, a third interface 158, and a fourth interface 159. The memory 151 stores instructions which are executed by the processor 152.

The first interface 156, the second interface 157 and the third interface 158 may include ports, disk drives, wireless antennas, or other types of receiver circuitry. The fourth interface 159 may be or include a user interface such as a touch-screen, a mouse, a keyboard, a microphone and/or a speaker. One or more of the first interface 156, the second interface 157 and the third interface 158 may connect, for example, the computer 110 to the display 180 in FIG. 1A or the controller 150 to the display 180 in FIG. 1B.

The controller 150 may perform some of the operations described herein directly and may implement other operations described herein indirectly. For example, the controller 150 may directly execute logical operations and indirectly control other operations such as by generating and transmitting content to be displayed on the display 180. Accordingly, the processes implemented by the controller 150 when the processor 152 executes instructions from the memory 151 may include steps not directly performed by the controller 150.

The processes implemented by the system 100, the device 101 and/or the controller 150 may include obtaining an ultrasound frame of a lung, identifying a pleural line in the lung, quantifying the pleural line, comparing the quantification of the pleural line to a predetermined value, and identifying pleural line abnormalities as important clinical features for COVID and other lung pathologies. The system 100, the device 101 and/or the controller may provide an interpretable assessment of the extent of pleural line abnormality. The outputs may be clearly visualized on the display 180, such as when the display 180 is provided for an ultrasound system, allowing clinicians to make their own clinical judgments and incorporate the information of pleural line abnormality into their overall decision-making process. The outputs include an output of indication of the at least one of irregularity or thickening in the pleural line. The output of the indication indication(s) may include outputting the indication alone as an output of data without the ultrasound frame on the display 180, as may include outputting the indication as data superimposed on the ultrasound frame on the display 180, or as data provided in one window separate from the ultrasound frame displayed in another window on the display 180. The indications may reflect one or both of irregularity or thickening when either or both are identified.

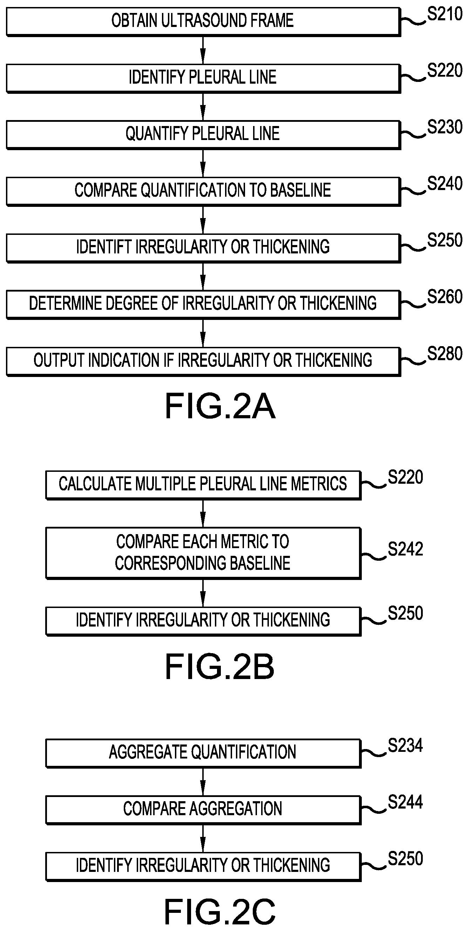

FIG. 2A illustrates a method for automated pleural line assessment in lung ultrasound, in accordance with a representative example for illustration purposes and not within the scope of the claims. The method of FIG. 2 may be performed by the system 100, by the device 101 or by the controller 150.

At S210, an ultrasound frame is obtained, such as by the computer 110 or the device 101. The ultrasound frame may be obtained by the computer 110 or the device 101 in real-time from an ultrasound imaging system as an ultrasound is performed. Alternatively, the ultrasound frame may be obtained by the computer 110 or the device 101 after the ultrasound is performed.

At S220, a pleural line is obtained based on the ultrasound frame. The pleural line may be obtained by the controller 150 applying image analysis to the ultrasound frame. The image analysis may include applying a trained artificial intelligence model, such as to detect the pleural line. The pleural line may be obtained automatically, may be segmented, and may be tracked such as when the pleural line is repeatedly obtained in real-time for each of a series of ultrasound frames during an ultrasound procedure. The pleural line may be automatically obtained, segmented, and tracked as an input to the assessment described herein. Once the pleural line is initially detected, a tracking algorithm may be applied for subsequent ultrasound frames to minimize the image processing burden for the subsequent frames, such as by minimizing the number of pixels to be analyzed repeatedly to find the pleural line if the pleural line had to be independently detected in each ultrasound frame.

The pleural line may be obtained at S220 based on detecting ultrasound imaging of the lung, such as by receiving an ultrasound image frame and detecting that the ultrasound image frame is of the lung. The computer 110 or the device 101 may automatically search for the pleural line, such as by using a trained artificial intelligence model that detects pleural lines in ultrasound frames of the lung. A software program may be automatically executed to perform S230, S240, S250, S260, and S280 based on confirming the pleural line at S220.

At S230, the pleural line is quantified. The quantification may be performed automatically by the controller 150, and may include counting the numbers of pixels that include the pleural line in each column and row of the display 180. The pleural line may be segmented before or at S230, so that the quantification is performed based on the segmented pleural line. As used herein, segmenting a pleural line or segmentation of a pleural line can refer to identification of pixels in an image that are part of a particular object. For example, segmentation may be used to identify a pleural line or parts of a pleural line. In another example, segmentation may be used to identify a boundary of an object and further may be used to identify that pixels on a particular side of a boundary are part of an object. In an example, when referring to a segmented pleural line, the pixels in an image that have been identified to be on one side of an identified boundary may be part of the pleural line structure. Having the segmentation of a pleural line, in other words, knowing the image pixels that constitute the pleural line, allows quantification of the shape, size, texture, and other characteristics of the pleural line.

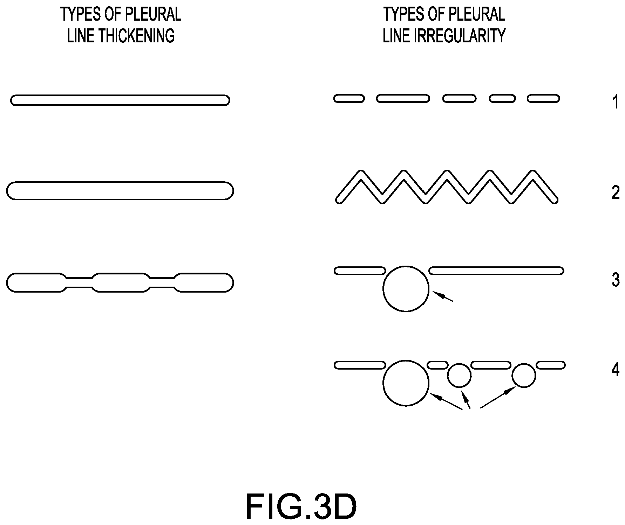

The number of pixels that include the pleural line in a column may reflect the thickness of the pleural line. The quantification at S230 may also include assigning coordinates to the pixels that include the pleural line, so that geometric discontinuity or other forms of irregularity may be determined from geometric patterns of pixels that include or that do not include the pleural line. Forms of irregularity are illustrated in FIG. 3D, including the geometric discontinuity illustrated as example (1). Other forms of irregularity may include the absence of smoothness shown as example (2) or a variation in intensity. Smoothness may be determined from the quantification at S230 based on an amount that edges of the pleural line deviate from a best fit line through the pleural line. Yet other forms of irregularity may include ultrasound features detected adjacent to the pleural line shown as example (3) or interrupting the pleural line as shown as example (4). These may include consolidations or the origin of B-lines that intersect the pleural line. The presence, quantity, or appearance of these other features may be used to derive the extent of pleural line irregularity.

Quantification of the pleural line at S230 may also include extracting additional image features such as overall pleural line brightness/intensity, homogeneity, fractal dimension and speckle/texture statistics. These image features may be derived based on image processing, or based on a supervised machine learning approach. Supervised machine learning may be more accurate but may require more labelled data.

At S240, the quantification is compared to a predetermined value that may characterize a normal, healthy pleural line. The comparison at S240 may be performed by the controller 150. The predetermined value may be set as a threshold to identify irregularity or thickening, and may be used for multiple pleural line assessments. The comparison at S240 may include comparing multiple different pleural line metrics to different predetermined values. As explained elsewhere herein, comparisons may also be made for aggregations of quantifications, either for different metrics or for different zones of the pleural line, so that aggregations of one or more quantified metrics and/or zones are compared to one or more relevant predetermined value(s).

At S250, thickening and/or irregularity is/are identified. The identification at S250 may be performed by the controller 150. quantifying an irregularity identified in the pleural line.

### Thickening

Pleural line thickness may be estimated from the quantification at S230. Pleural thickness may reflect mean thickness, maximum thickness, thickness variability and/or another aspect of thickness that contributes to the overall thickness of the pleural line. For example, a full width half max (FWHM) in the axial direction may be calculated for each pixel along the pleural line. Overall thickness may be the mean of all FWHM measurements. Maximum thickness may, for example, be the highest quartile of all FWHM measurements for the pleural line. The process of estimating pleural line thickness may also include a pre-processing step before the FWHM calculation to highlight the pleural line, such as by applying an image filter such as a curvilinear filter. In some embodiments, pleural line thickness may be obtained from direct segmentation of the full pleural line boundary, for example using supervised machine learning methods. Direct segmentation of the full pleural line boundary may improve accuracy at the expense of greater processing requirements for larger amounts of labelled data.

In some embodiments, additional measurements related to thickness may be assessed. Such additional measurements may include uniformity or variation of the thickness across the pleural line, with higher variation in pleural thickness indicative of increased irregularity.

### Irregularity

The identification at S250 may also or alternatively include an automatic assessment of pleural line irregularity. Irregularity may include at least one measure of discontinuity, smoothness, or variation in intensity. The measure of discontinuity may reflect discontinuity/interruptions in the pleura line. The measure of smoothness may be a measure of the absence of smoothness. The measure of the variation in intensity may be a measure of variations in brightness/intensity in pixels that include the pleural line.

In some embodiments, a discontinuity index may be used to assess the degree of pleural line discontinuity/interruptions. Pleural line discontinuity/interruptions may reflect quantity/distribution of interrupted pleural line segments, curve fitting followed by counting of pleural line segments, and/or another aspect of discontinuity that reflects overall discontinuity. A pathological pleural line (including in COVID-19 pneumonia) may be discontinuous, with islands of smooth and high-intensity pleural line visible, but interrupted by segments of poorly visible, lower-intensity or disappearing pleural line. One method to assess the level of discontinuity is to threshold a narrow band around the pleural line to the highest quartile of intensity values in that region, and count the number of individual high-intensity islands that emerge. Spatial smoothing before thresholding or rejection of single-pixel islands can be used to remove noise from the measurement. An alternative method involves fitting a low-order polynomial or spline to the pleural line, and counting the number of continuous segments along that curve for which intensity values are above a threshold (e.g. highest quartile).

In some embodiments, a spatial/geometric irregularity index may be used to assess the spatial smoothness of the pleural line. Pleural line spatial/geometric irregularity may reflect spatial variability/tortuosity or homogeneity, spatial frequency, spatial derivatives or curvature statistics, and/or another aspect of spatial geometric irregularity that reflects overall spatial/geometric irregularity. A pathological pleural line will show higher spatial variance and be more jagged, while a healthy pleural line is more likely to fall along a straight line and thus have lower spatial variance and be smoother. Any number of methods may be used to capture the smoothness or tortuosity. As one example, the number of first derivative changes along the pleural line, which may be calculated after applying a spatial low pass filter to avoid noisy fluctuations in the profile, may be used to capture smoothness. Alternatively a spatial frequency approach may be used to pick up the power in high frequency bands in the spatial spectrum of the pleural line contour. Another approach that may be used may compute the index of the maximum-intensity pixel along each column of the pleural line and then calculate the variance of these maximum-intensity positions.

Pleural line brightness/intensity may reflect mean brightness and/or intensity, maximum brightness/intensity, a percentile threshold such as 95th percentile brightness/intensity, brightness/intensity variability or homogeneity, texture/speckle statistics or intensity derivatives, and/or another aspect of brightness/intensity that reflects the overall brightness/intensity of the pleural line. In some embodiments, an intensity-based irregularity index may be used for the quantification to assess changes in pixel intensity along the pleural line. A healthy pleural line will show lower intensity variance which shows more smoothness. Pathological pleural lines will be characterized by higher intensity variance which shows lower smoothness and more irregularity and discontinuity. A simple metric that may be used to capture smoothness is the standard deviation, normalized to mean, of the pleural line intensity values.

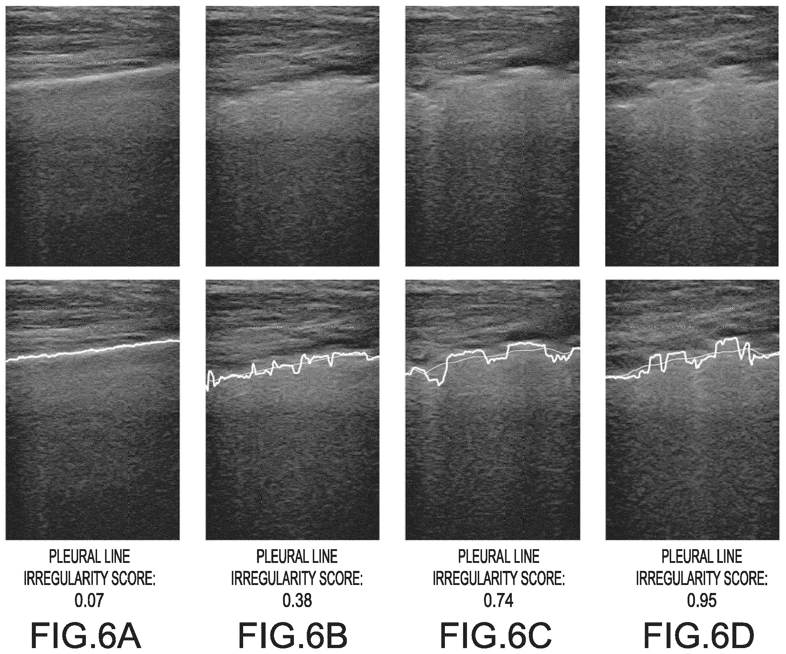

As yet another approach, rather than variance, the mean squared error or mean absolute error between the maximum-intensity positions and a smoothed pleural line projection may be calculated. The results of this approach are shown in and described with respect to FIG. 6A, FIG. 6B, FIG. 6C and FIG. 6D.

Yet another approach may include detecting ultrasound features located adjacent to the pleural line or interrupting the pleural line. These features could include consolidations or the origin of B-lines that intersect the pleural line. The presence, quantity, or appearance of these other features may be used to derive the extent of pleural line irregularity. For example, the number of small subpleural consolidations and/or subpleural B-lines identified along the pleural line may be used as an indicator of the irregularity of the pleural line itself, to classify a normal or abnormal pleural line, or to determine a pleural line severity score.

At S260, a degree of irregularity and/or thickening is determined. The degree may reflect a wide measurement such as A, B, C or D, to broadly reflect the relative severity and potential ramifications of the irregularity and/or thickening. Alternatively, the degree may reflect a quantification such as a percentile or numerical score from 1 to 100. The degree may reflect a score of the severity and potential ramifications of irregularity or thickening. For example, irregularity or thickening may be quantified as a percentile relative to a broad cross section of pleural lines in ultrasounds subject to the process of FIG. 2A. The determination at S260 may be, for example, a determination of a severity level associated with the pleural line, a determination of the pleural line as being either normal or abnormal, a determination based on quantifying the pleural line as an assessment of an overall condition of the lung, and/or a determination based on quantifying the pleural line a likelihood of one or more pathological conditions. The degree or severity may be determined based on metrics of the image features extracted from the ultrasound frame and showing irregularity of the pleural line.

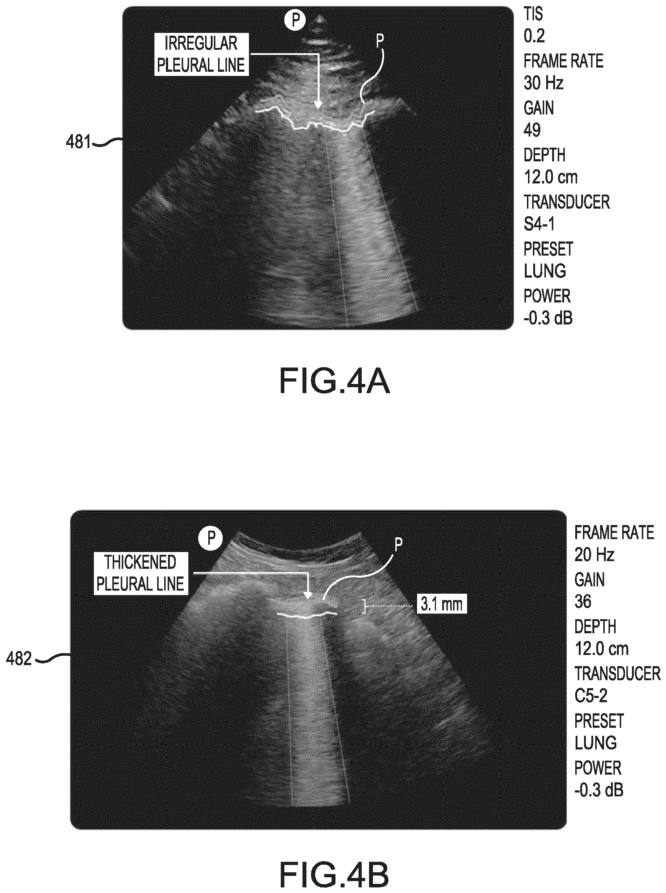

At S280, an indication of irregularity and/or thickening is output. For example, the computer 110 may output an indication of the at least one of irregularity or thickening in the pleural line. The output at S280 may be via the display 180, and may indicate a visualization that includes a clinical prediction of pleural line abnormality. The indication is output as data superimposed on the ultrasound frame of the lung on the display 180. The indication may be a set of numerical scores or a visual overlay on top of the ultrasound frame. As an example, highly thickened or irregular pleural lines may have a differently colored overlay compared to healthy/normal pleural lines, as described with respect to FIG. 4A and FIG. 4B.

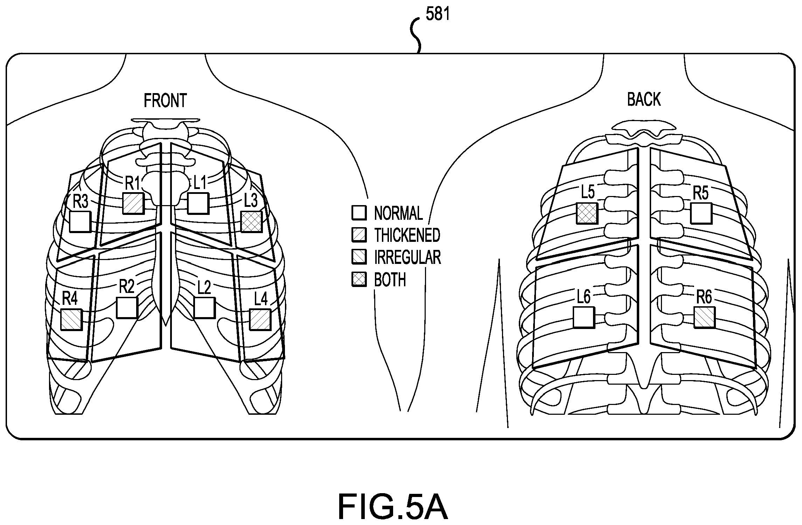

A visual summary screen or dashboard 581 shown in FIG. 5A may also be employed to provide a high level overview of pleural line abnormality across all lung zones of an exam. Similar visualizations may be employed to display pleural line information across multiple lung ultrasound exams carried out on a patient, e.g. to facilitate monitoring and disease management.

FIG. 2B illustrates another method for automated pleural line assessment in lung ultrasound, in accordance with a representative example for illustration purposes and not within the scope of the claims.

At S232, multiple pleural line metrics are calculated. Pleural line metrics may include metrics that reflect physical characteristics of the pleural line, including thickness at one or more points along the pleural line, discontinuity anywhere along the pleural line, relative smoothness of edges of the pleural line, and more.

At S242, each metric is compared to a corresponding predetermined value. Predetermined values may be derived from clinical standards, and standardized for application in the automated pleural line assessment in lung ultrasound as described herein.

At S250, irregularity or thickening is identified. Irregularity or thickening may be identified based on the comparison to the predetermined value at S242, and may lead to the determination of the degree of irregularity and/or thickening at S260.

FIG. 2C illustrates another method for automated pleural line assessment in lung ultrasound, in accordance with a representative example for illustration purposes and not within the scope of the claims.

At S234, quantification is aggregated. One or more quantified metrics may be aggregated across a lung ultrasound video or different consecutive or even non-consecutive frames in the lung ultrasound video. One or more metrics may also be aggregated and scored across lung zones, for a single frame or for different consecutive or even non-consecutive frames in the lung ultrasound video.

In some embodiments, lung zones may be equal-weighted in the scoring. In other embodiments, lung zones may be differentially-weighted, such as based on proximity to an identified area of problematic tissue, or based on proximity to a predetermined location in the lungs. In some embodiments, an indication score for one lung zone may influence an individual score for another lung zone, such as based on the one lung zone containing an identified discontinuity. If certain lung zones are known to be more correlated to relevant clinical measurements - such as oxygen level or a differential diagnosis of a particular condition e.g. pneumonia or pulmonary edema - those zones may be given higher weighting than the others. Similarly, if certain lung zones are scanned more regularly as part of simplified lung ultrasound protocols involving a reduced number of acquired lung zones, these zones may be given higher weighting.

The aggregated quantification of the pleural line may reflect scoring across a lung ultrasound video and may include pleural line measurements such as pleural thickness, intensity-based irregularity, and spatial irregularity. These measurements may be extracted from each frame of the ultrasound video, and then aggregated across the entire video to obtain an overall set of scores for the video. Uniformity or variation of the set of pleural line measurements across all frames in the video may be assessed. A high fluctuation of measurements/scores may indicate increased irregularity (higher variation0, whereas a low fluctuation may indicate less irregularity (lower variation).

Pleural line dynamics may also be extracted by examining a series of frames in the lung ultrasound video or by tracking pleural line feature metrics across the video. Pleural line dynamics may reflect a sum, average, variability, change, or other statistic derived from tracking thickness, brightness/intensity, spatial/geometric irregularity, and/or discontinuity across frames in an ultrasound video clip. Pleural line scores may be aggregated across all lung zones and exams over time, and may reflect the sum, average, variability, change and/or another statistic used to quantify and visualize and report thickness, brightness/intensity, spatial/geometric irregularity, and/or discontinuity across all lung zones in an exam. Pleural line scores may also reflect the sum, average, variability, change and/or another statistic used to quantify and visualize and report thickness, brightness/intensity, spatial/geometric irregularity, and/or discontinuity across multiple exams or time points.

These pleural line features and metrics may be extracted from each individual ultrasound frame, or from multiple frames of the video loop. For example, a rolling buffer of several continuous frames may be stored and updated with each incoming new frame, and this buffer may be continuously provided to the pleural line assessment algorithm to evaluate thickness and irregularity. Often in the case of ultrasound the use of multiple frames improves the accuracy of characterization compared to separately processing individual frames.

The aggregated scoring may also be performed across multiple lung zones, multiple exams, or multiple time points. An overall evaluation of pleural abnormality may be obtained by aggregating scores across all lung zones in one or more exam(s). Different lung zones may be given different weights in this overall aggregation step based on prior clinical data or knowledge.

At S244, the aggregation is compared. The aggregation(s) from S234 may be compared to one or more predetermined value(s), and the comparison may provide an improved result in that the comparison reflects metrics of the pleural line from multiple frames so that a deviation in any particular frame is less likely to result in a misidentification of irregularity or thickening or a lack thereof.

At S250, irregularity or thickening is identified. The image processing, quantification and scoring may be used for an overall clinical prediction of pleural abnormality. For example, the quantified metrics may be combined into a single clinical evaluation of pleural abnormality, such as a unified clinical score for pleural lines. A prediction model trained by the AI training system 195 may be refined against clinical annotations of healthy versus abnormal pleural lines. Alternatively, a prediction model trained by the AI training system 195 may be developed to directly assess a disease state, such as COVID-19 pneumonia or other respiratory condition. The overall clinical predictive value of the automated pleural line feature extraction model may be determined based on validation of the model on data from unseen clinical subjects.

The information displayed to the end-user may come in any number of forms, including: a simple binary indication of normal versus abnormal pleural line for each scanned lung zone; a severity score (e.g. 0, 1, 2, 3) indicating a degree of pleural line severity or pathology; or a descriptive/categorical assessment such as pleural line thickening and pleural line irregularity.

In the first two scenarios, the "abnormal" value and the severity score may take into account descriptive visual metrics such as thickening and irregularity. In the case of image processing, these descriptive visual metrics might be extracted as image features. In the case of a machine learning or artificial intelligence approach, the metrics might be defined in the training labels used to refine the model. Either way, it is possible that this underlying information is not necessarily shown to the user.

The pleural line result may be directly provided to the end user, or may be incorporated into a more comprehensive evaluation, such as an overall lung severity score that takes into account other lung ultrasound features in addition to the pleural line. In this scenario, the user would not see the explicit pleural line output, but rather the overall measurement.

The image processing, quantification and scoring may employ a trained artificial intelligence model learned directly using machine/deep learning artificial intelligence frameworks. The trained artificial intelligence model may be applied to the quantification of the pleural line to compare the quantification(s) of the pleural line to the predetermined value(s) and to identify at least one of irregularity and thickening. Alternatively, hybrid approaches may be employed combining engineered image features and learning approaches. Artificial intelligence methods may be employed as an initialization step to locate the pleural line, and then followed by the feature extraction, feature learning, and classification steps detailed here. Similarly, image processing methods may be employed to detect the pleural line, and artificial intelligence methods may be applied to characterize the pleural line as normal, thickened, irregular, or otherwise.

Detected pleural lines may be shown on screen during live scanning or when reviewing previously saved video loops. The display could be in the form of bounding boxes, arrows, segmentation outlines, or other image overlays. The category of pleural line could be shown in the overlay for example in different colors (e.g. green = normal, yellow/orange/red = abnormal/thickened/irregular) or other visualization techniques.

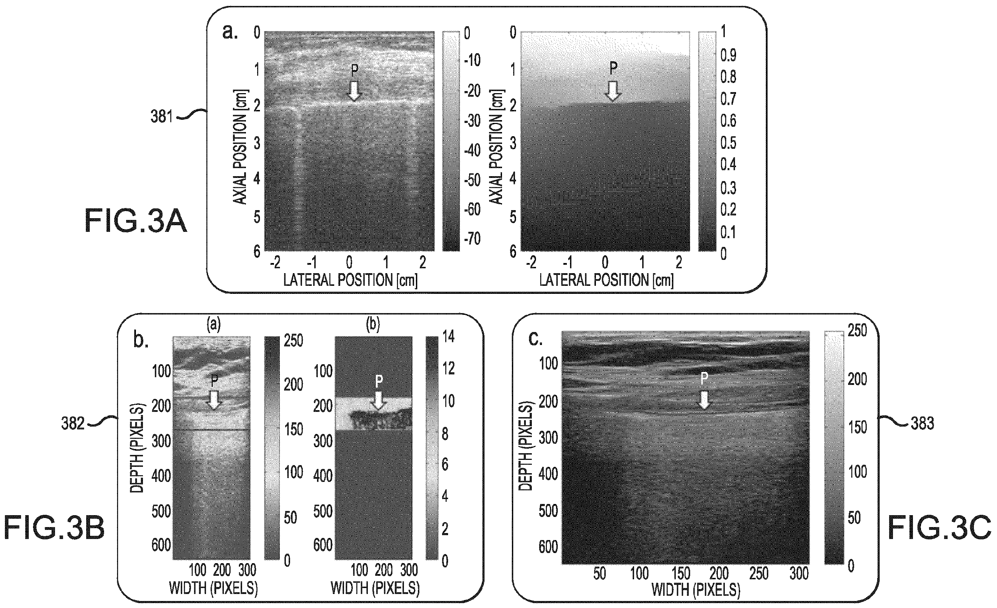

FIG. 3A illustrates a visualization of a pleural line detected using an image processing algorithm, in accordance with a representative embodiment.

In FIG. 3A, image processing is applied to compute a confidence map to detect and visualize likely boundaries of a pleural line P designated by the arrow. The confidence map in FIG. 3A is displayed on screen 381. The confidence map indicates likely pleural boundaries based on lung motion. The image processing reflected in FIG. 3A may result in obtaining the pleural line at S220 in FIG. 2A.

FIG. 3B illustrates a visualization of peak detection signal processing and a lung sliding motion map, in accordance with a representative embodiment.

In FIG. 3B, algorithms applied to detect and visualize a pleural line P designated by the arrow. The algorithms include peak detection signal processing to generate a lung sliding motion map. The lung sliding motion map is displayed on a screen 382. The lung sliding motion map is computed from a small buffer of frames. The algorithms reflected in FIG. 3B may result in obtaining the pleural line at S220 in FIG. 2A.

FIG. 3C illustrates a visualization of a segmented pleural line, in accordance with a representative embodiment.

The segmented pleural line P in FIG. 3C is designated by the arrow and may be the pleural line obtained at S220 in FIG. 2A. The segmented pleural line P in FIG. 3C is displayed on a screen 383.

Although not illustrated in FIG. 3A, FIG. 3B or FIG. 3C, a deep learning based pleural line detection approach may also be used to detect and visualize a pleural line. The deep learning based approach may use a convolutional neural network (CNN) as a trained artificial intelligence model. The teachings herein extract clinically relevant features from the pleural line, independent of how the pleural line itself is detected and made visualizable in FIG. 3A, FIG. 3B, FIG. 3C or any other mechanisms for detecting pleural lines including using trained artificial intelligence models to detect pleural lines.

FIG. 4A illustrates a visualization of an irregular pleural line, in accordance with a representative embodiment. FIG. 4A illustrates pleural line irregularity on a lung ultrasound screen 481 displayed on, for example, the display 180. Irregularity may include any of discontinuity, the absence of smoothness, variations in intensity, or other forms of deviations that may be detected and quantified in digital images of the pleural line. The irregular pleural line P is designated by an arrow, and may be displayed in color for segments of the pleural line P which are irregular.

FIG. 4B illustrates a visualization of a thickened pleural line, in accordance with a representative embodiment.

FIG. 4B illustrates pleural line thickness on a lung ultrasound screen 482 displayed on, for example, the display 180. Pleural line thickness may reflect a count of pixels that include the pleural line in an image on the display 180, or any of the variety of metrics described above which can be used as thickness descriptors. The thickened pleural line P is designated by an arrow, and may be displayed in color for segments of the pleural line P which are thickened.

FIG. 5A illustrates a dashboard for automated pleural line assessment in lung ultrasound, in accordance with a representative embodiment.

In FIG. 5A, the visual summary screen or dashboard 581 provides a high level overview of pleural line abnormality across the lung zones of an exam, and may be displayed on the display 180. The dashboard 581 in FIG. 5A includes front and back views of lung zones, including eight lung zones (L1 to L4 and R1 to R4) on the front view and four lungs zones (L5 to L6 and R5 to R6) on the back view. A key is provided in the dashboard 581 of FIG. 5A to indicate the label for each of the eight lung zones on the front view and the four lung zones on the back view. The four possible labels include Normal, Thickened, Irregular, or Both.

The user may evaluate the pleural line result shown in each lung zone to support clinical decision-making. For example, the user may look for bilateral pleural line abnormalities (i.e. zones with abnormal pleural lines on both the left lung and right lung, or both anterior and posterior). One may also look for concentrated regions with heavy abnormality, for example many abnormal zones in the lower right lung. It is possible also to ignore the distribution and focus simply on the sum or average across the zones. Finally, one might evaluate changes in pleural line status over time, e.g. during a patient's extended hospital stay, as a method of longitudinal monitoring.

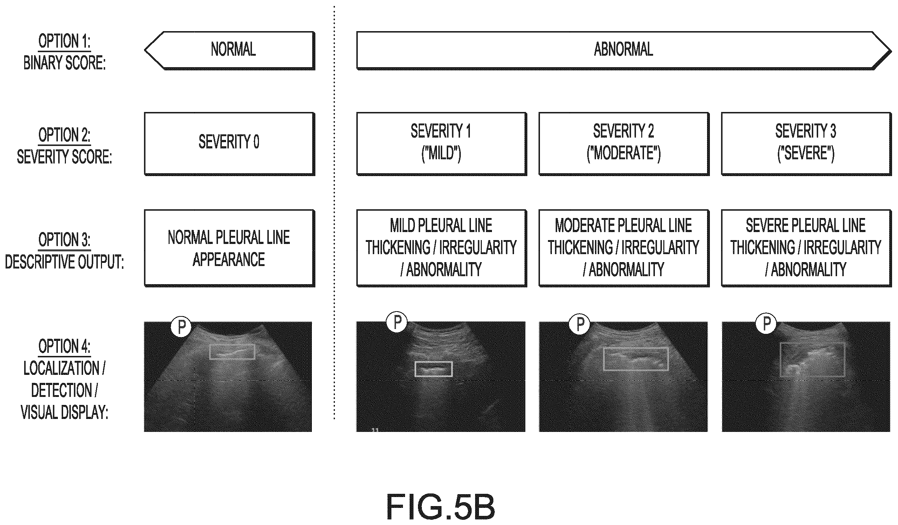

FIG. 5B illustrates output options for the dashboard shown in FIG. 5A, in accordance with a representative embodiment.

In FIG. 5B, output options that may be output via the dashboard 581 in FIG. 5A include a binary score (Option 1) for normal/abnormal score, a severity score (Option 2) for 0/1/2/3, a descriptive output (Option 3) for normal/thickened/irregular, and a visual output (Option 4) for the pleural line frame localization/detection. Output of characterizations of irregularity or thickening are not limited to visualizations or these types of visualizations. Instead, characterizations of irregularity or thickening may also be output audibly, or selectively only if the result warrants attention from a clinician, or otherwise.

FIG. 6A illustrates an automatically-detected pleural line and corresponding quantifications, in accordance with a representative embodiment. FIG. 6B illustrates another automatically-detected pleural line and corresponding quantifications, in accordance with a representative embodiment. FIG. 6C illustrates another automatically-detected pleural line and corresponding quantifications, in accordance with a representative embodiment. FIG. 6D illustrates another automatically-detected pleural line and corresponding quantifications, in accordance with a representative embodiment.

FIG. 6A, FIG. 6B, FIG. 6C and FIG. 6D show calculations of pleural line abnormality which are based on mean squared error or mean absolute error between the maximum-intensity positions and a smoothed pleural line projection. FIG. 6A, FIG. 6B, FIG. 6C and FIG. 6D show examples of automated pleural line irregularity index scores. In FIG. 6A, the pleural irregularity score is 1.307, the lowest among any of FIG. 6A, FIG. 6B, FIG. 6C and FIG. 6D. In FIG. 6B, the pleural irregularity score is 5.122, the second lowest among any of FIG. 6A, FIG. 6B, FIG. 6C and FIG. 6D. In FIG. 6C, the pleural irregularity score is 9.554, the second highest among any of FIG. 6A, FIG. 6B, FIG. 6C and FIG. 6D. In FIG. 6D, the pleural irregularity score is 12.362, the highest among any of FIG. 6A, FIG. 6B, FIG. 6C and FIG. 6D.

The implementation shown in FIG. 6A, FIG. 6B, FIG. 6C and FIG. 6D may be carried out in data visualization software. First, a smoothed curvilinear segmentation of the pleural line may be estimated. From this, the pixel coordinate with the maximum intensity either above or below the smoothed pleural line segmentation may be calculated. The root mean squared offset between the segmentation line and maximum intensity line may be computed along the horizontal direction to produce "pleural irregularity scores" for each image frame. As seen, lower scores correspond with a smoother pleural appearance, and high scores correspond with a more irregular appearance. For FIG. 6A, FIG. 6B, FIG. 6C and FIG. D, an algorithm for data visualization software may be used to implement these displays including the irregularity scores. The algorithm achieves automated pleural line detection and automated quantification of a pleural irregularity index.

FIG. 7 illustrates a computer system, on which a method for automated pleural line assessment in lung ultrasound is implemented, in accordance with another representative embodiment.

FIG. 7 illustrates a computer system, on which a method for automated pleural line assessment in lung ultrasound is implemented, in accordance with another representative embodiment.

Referring to FIG.7, the computer system 700 includes a set of software instructions that can be executed to cause the computer system 700 to perform any of the methods or computer-based functions disclosed herein. The computer system 700 may operate as a standalone device or may be connected, for example, using a network 701, to other computer systems or peripheral devices. In embodiments, a computer system 700 performs logical processing based on digital signals received via an analog-to-digital converter.

In a networked deployment, the computer system 700 operates in the capacity of a server or as a client user computer in a server-client user network environment, or as a peer computer system in a peer-to-peer (or distributed) network environment. The computer system 700 can also be implemented as or incorporated into various systems or devices, such as an ultrasound system, a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The computer system 700 can be incorporated as or in a device that in turn is in an integrated system that includes additional devices. In an embodiment, the computer system 700 can be implemented using electronic devices that provide voice, video, or data communication. Further, while the computer system 700 is illustrated in the singular, the term "system" shall also be taken to include any collection of systems or sub-systems that individually or jointly execute a set, or multiple sets, of software instructions to perform one or more computer functions.

As illustrated in FIG. 7, the computer system 700 includes a processor 710. The processor 710 may be considered a representative example of a processor of a controller and executes instructions to implement some or all aspects of methods and processes described herein. The processor 710 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 710 is an article of manufacture and/or a machine component. The processor 710 is configured to execute software instructions to perform functions as described in the various embodiments herein. The processor 710 may be a general-purpose processor or may be part of an application specific integrated circuit (ASIC). The processor 710 may also be a microprocessor, a microcomputer, a processor chip, a controller, a microcontroller, a digital signal processor (DSP), a state machine, or a programmable logic device. The processor 710 may also be a logical circuit, including a programmable gate array (PGA), such as a field programmable gate array (FPGA), or another type of circuit that includes discrete gate and/or transistor logic. The processor 710 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device including "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 700 further includes a main memory 720 and a static memory 730, where memories in the computer system 700 communicate with each other and the processor 710 via a bus 708. Either or both of the main memory 720 and the static memory 730 may be considered representative examples of a memory of a controller, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 720 and the static memory 730 are articles of manufacture and/or machine components. The main memory 720 and the static memory 730 are computer-readable mediums from which data and executable software instructions can be read by a computer (e.g., the processor 710). Each of the main memory 720 and the static memory 730 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 700 further includes a video display unit 750, such as a liquid crystal display (LCD), an organic light emitting diode (OLED), a flat panel display, a solid-state display, or a cathode ray tube (CRT), for example. Additionally, the computer system 700 includes an input device 760, such as a keyboard/virtual keyboard or touch-sensitive input screen or speech input with speech recognition, and a cursor control device 770, such as a mouse or touch-sensitive input screen or pad. The computer system 700 also optionally includes a disk drive unit 780, a signal generation device 790, such as a speaker or remote control, and/or a network interface device 740.

In an embodiment, as depicted in FIG. 7, the disk drive unit 780 includes a computer-readable medium 782 in which one or more sets of software instructions 784 (software) are embedded. The sets of software instructions 784 are read from the computer-readable medium 782 to be executed by the processor 710. Further, the software instructions 784, when executed by the processor 710, perform one or more steps of the methods and processes as described herein. In an embodiment, the software instructions 784 reside all or in part within the main memory 720, the static memory 730 and/or the processor 710 during execution by the computer system 700. Further, the computer-readable medium 782 may include software instructions 784 or receive and execute software instructions 784 responsive to a propagated signal, so that a device connected to a network 701 communicates voice, video, or data over the network 701. The software instructions 784 may be transmitted or received over the network 701 via the network interface device 740.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, automated pleural line assessment in lung ultrasound enables an automated system and method for detection and quantification of pleural line changes derived from ultrasound imaging systems. The automated pleural line assessment may be used in emergency cases of acute respiratory diseases or thoracic diseases. The system may also be used in (a) pre-hospital settings, (b) initial evaluation in the emergency room, (c) monitoring during treatment (including in a critical care setting), and (d) follow-up after proper treatments, and is applicable to many ultrasound imaging systems including point-of-care applications. The system may be used in ambulances, emergency rooms (ERs), critical care environments, or surgery situations.

Although automated pleural line assessment in lung ultrasound has been described with reference to several exemplary embodiments, it is understood that the words that have been used are words of description and illustration, rather than words of limitation. Changes may be made within the purview of the appended claims, as presently stated and as amended, without departing from the scope and spirit of automated pleural line assessment in lung ultrasound in its aspects. Although automated pleural line assessment in lung ultrasound has been described with reference to particular means, materials and embodiments, automated pleural line assessment in lung ultrasound is not intended to be limited to the particulars disclosed; rather automated pleural line assessment in lung ultrasound extends to many functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The preceding description of the disclosed embodiments is provided to enable any person skilled in the art to practice the concepts described in the present disclosure. As such, the above disclosed subject matter is to be considered illustrative, and not restrictive. Thus, to the maximum extent allowed by law, the scope of the present disclosure is to be determined by the broadest permissible interpretation of the following claims and their equivalents and shall not be restricted or limited by the foregoing detailed description.

## Claims

1. A tangible non-transitory computer readable storage medium (782) that stores a computer program (784), wherein the computer program (784), when executed by a processor (152, 710), causes a computer apparatus (110, 700) to perform the steps of a method comprising:
obtaining (S210) an ultrasound frame of a lung and identifying (S220) a pleural line in the lung;
quantifying (S230) the pleural line identified in the lung to obtain a quantification of the pleural line;
comparing (S240) the quantification of the pleural line to a predetermined value that characterizes a normal, healthy pleural line; and
identifying (S250) at least one of irregularity or thickening in the pleural line based on comparing the quantification of the pleural line to the predetermined value,
**characterized in that** it further comprises outputting (S280) an indication of the at least one of irregularity or thickening in the pleural line,
wherein the indication is output as data superimposed on the ultrasound frame on a display (180, 750).

2. The tangible non-transitory computer readable storage medium (782) of claim 1, wherein the method further comprises the step of:
determining (S260) a degree of the at least one of irregularity or thickening in the pleural line based on the quantification of the pleural line.

3. The tangible non-transitory computer readable storage medium (782) of claim 2, wherein determining (S260) the degree of the at least one of irregularity or thickening in the pleural line comprises at least one of:
- determining a severity level associated with the pleural line;
- determining a pleural line as being either normal or abnormal;
- determining, based on the quantification of the pleural line, an assessment of an overall condition of the lung; or
- determining, based on the quantification of the pleural line, a likelihood of one or more pathological conditions.

4. The tangible non-transitory computer readable storage medium (782) of claim 2,
wherein the computer program (784), when executed by a processor (152, 710), further causes the computer apparatus (110, 700) to extract image features showing irregularity in the pleural line, and
wherein determining (S260) the degree of the irregularity is based on metrics of the image features.

5. The tangible non-transitory computer readable storage medium (782) of claim 1, wherein the method further comprises the step of:
quantifying an irregularity identified in the pleural line, wherein the irregularity comprises at least one measure of discontinuity, smoothness, or variation in intensity.

6. The tangible non-transitory computer readable storage medium (782) of claim 1, wherein the method further comprises the step of:
quantifying thickening identified in the pleural line, wherein the thickening comprises at least one measure of a width of the pleural line, thickness of a segmentation of the pleural line, or area of the segmentation of the pleural line.

7. The tangible non-transitory computer readable storage medium (782) of claim 1, wherein the quantifying (S230) further comprises:
calculating (S232) a plurality of pleural line metrics.

8. The tangible non-transitory computer readable storage medium (782) of claim 7, wherein the comparing (S240) further comprises:
comparing (S242) each of the plurality of pleural line metrics to a corresponding predetermined value.

9. The tangible non-transitory computer readable storage medium (782) of claim 1, wherein the method further comprises the step of:
detecting ultrasound imaging of the lung; and
causing the computer apparatus (110, 700) to automatically perform the method based on detecting the ultrasound imaging of the lung.

10. The tangible non-transitory computer readable storage medium (782) of claim 1, wherein the method further comprises the step of:
aggregating (S234) the quantification of the pleural line identified in the lung from the ultrasound frame with another quantification of the pleural line identified in the lung from another ultrasound frame to obtain an aggregated quantification of the pleural line, and
identifying (S250) at least one of irregularity or thickening in the pleural line based on comparing (S244) the aggregated quantification of the pleural line to the predetermined value.

11. A system (100) for processing an ultrasound frame, comprising:
an ultrasound controller (150) comprising a memory (151) that stores instructions and a processor (152) that executes the instructions, wherein, when executed by the processor (152), the instructions cause the ultrasound controller (150) to:
obtain (S210) an ultrasound frame of a lung and identify (S220) a pleural line in the lung;
quantify (S230) the pleural line identified in the lung to obtain a quantification of the pleural line;
compare (S240) the quantification of the pleural line to a predetermined value; and
identify (S250) at least one of irregularity or thickening in the pleural line based on comparing the quantification of the pleural line to the predetermined value,
**characterized in that** the instructions further cause the ultrasound controller (150) to output (S280) an indication of the at least one of irregularity or thickening in the pleural line,
wherein the indication is output as data superimposed on the ultrasound frame.

12. The system (100) of claim 11, further comprising:
a display (180) that displays the indication of the at least one of irregularity or thickening in the pleural line.

13. The system (100) of claim 11, wherein, when executed by the processor (152), the instructions cause the ultrasound controller (150) further to:
apply a trained artificial intelligence model to the quantification of the pleural line to compare (S240) the quantification of the pleural line to the predetermined value and to identify (S250) at least one of irregularity or thickening.

14. The system (100) of claim 11, wherein, when executed by the processor (152), the instructions cause the ultrasound controller (150) further to:
quantify an irregularity identified in the pleural line, wherein the irregularity comprises at least one measure of discontinuity, smoothness, or variation in intensity.

15. The system (100) of claim 11, wherein, when executed by the processor (152), the instructions cause the ultrasound controller (150) further to:
quantify thickening identified in the pleural line, wherein the thickening comprises at least one measure of a width of the pleural line, thickness of a segmentation of the pleural line, or area of the segmentation of the pleural line.

## Patentansprüche

1. Materielles, nicht-transitorisches, computerlesbares Speichermedium (782), das ein Computerprogramm (784) speichert, wobei das Computerprogramm (784), wenn es von einem Prozessor (152, 710) ausgeführt wird, eine Computereinrichtung (110, 700) veranlasst, die Schritte eines Verfahrens durchzuführen, umfassend:
Erhalten (S210) eines Ultraschalleinzelbildes einer Lunge und Identifizieren (S220) einer Pleuralinie in der Lunge;
Quantifizieren (S230) der in der Lunge identifizierten Pleuralinie, um eine Quantifizierung der Pleuralinie zu erhalten;
Vergleichen (S240) der Quantifizierung der Pleuralinie mit einem vorbestimmten Wert, der eine normale, gesunde Pleuralinie kennzeichnet; und
Identifizieren (S250) mindestens einer von Unregelmäßigkeit oder Verdickung in der Pleuralinie auf Basis des Vergleichens der Quantifizierung der Pleuralinie mit dem vorbestimmten Wert,
**dadurch gekennzeichnet, dass** es ferner Ausgeben (S280) einer Angabe der mindestens einen von Unregelmäßigkeit oder Verdickung in der Pleuralinie umfasst,
wobei die Angabe als Daten, die dem Ultraschalleinzelbild überlagert sind, auf einer Anzeige (180, 750) ausgegeben wird.

2. Materielles, nicht-transitorisches, computerlesbares Speichermedium (782) nach Anspruch 1, wobei das Verfahren ferner den folgenden Schritt umfasst:
Bestimmen (S260) eines Grades der mindestens einen von Unregelmäßigkeit oder Verdickung in der Pleuralinie auf Basis der Quantifizierung der Pleuralinie.

3. Materielles, nicht-transitorisches, computerlesbares Speichermedium (782) nach Anspruch 2, wobei Bestimmen (S260) des Grades der mindestens einen von Unregelmäßigkeit oder Verdickung in der Pleuralinie mindestens eines umfasst von:
- Bestimmen eines Schweregrades, der mit der Pleuralinie verknüpft ist;
- Bestimmen, dass eine Pleuralinie entweder normal oder abnormal ist;
- Bestimmen einer Beurteilung eines Gesamtzustands der Lunge auf Basis der Quantifizierung der Pleuralinie; oder
- Bestimmen einer Wahrscheinlichkeit eines oder mehrerer pathologischer Zustände auf Basis der Quantifizierung der Pleuralinie.

4. Materielles, nicht-transitorisches, computerlesbares Speichermedium (782) nach Anspruch 2, wobei das Computerprogramm (784), wenn es von einem Prozessor (152, 710) ausgeführt wird, ferner die Computereinrichtung (110, 700) veranlasst, Bildmerkmale zu extrahieren, die Unregelmäßigkeit in der Pleuralinie zeigen, und
wobei Bestimmen (S260) des Grades der Unregelmäßigkeit auf Basis von Metriken der Bildmerkmale erfolgt.

5. Materielles, nicht-transitorisches, computerlesbares Speichermedium (782) nach Anspruch 1, wobei das Verfahren ferner den folgenden Schritt umfasst:
Quantifizieren einer in der Pleuralinie identifizierten Unregelmäßigkeit, wobei die Unregelmäßigkeit mindestens ein Maß für Diskontinuität, Glätte oder Intensitätsvariation umfasst.

6. Materielles, nicht-transitorisches, computerlesbares Speichermedium (782) nach Anspruch 1, wobei das Verfahren ferner den folgenden Schritt umfasst:
Quantifizieren einer in der Pleuralinie identifizierten Verdickung, wobei die Verdickung mindestens ein Maß für eine Breite der Pleuralinie, Dicke einer Segmentierung der Pleuralinie oder Fläche der Segmentierung der Pleuralinie umfasst.

7. Materielles, nicht-transitorisches, computerlesbares Speichermedium (782) nach Anspruch 1, wobei das Quantifizieren (S230) ferner umfasst:
Berechnen (S232) einer Vielzahl von Pleuralinienmetriken.

8. Materielles, nicht-transitorisches, computerlesbares Speichermedium (782) nach Anspruch 7, wobei das Vergleichen (S240) ferner umfasst:
Vergleichen (S242) jeder der Vielzahl von Pleuralinienmetriken mit einem entsprechenden vorbestimmten Wert.

9. Materielles, nicht-transitorisches, computerlesbares Speichermedium (782) nach Anspruch 1, wobei das Verfahren ferner den folgenden Schritt umfasst:
Erkennen einer Ultraschallbildgebung der Lunge; und
Veranlassen der Computereinrichtung (110, 700), das Verfahren automatisch auf Basis eines Erkennens von Ultraschallbildern der Lunge durchzuführen.

10. Materielles, nicht-transitorisches, computerlesbares Speichermedium (782) nach Anspruch 1, wobei das Verfahren ferner den folgenden Schritt umfasst:
Aggregieren (S234) der Quantifizierung der in der Lunge anhand des Ultraschalleinzelbildes identifizierten Pleuralinie mit einer weiteren Quantifizierung der in der Lunge anhand eines anderen Ultraschalleinzelbildes identifizierten Pleuralinie, um eine aggregierte Quantifizierung der Pleuralinie zu erhalten, und
Identifizieren (S250) mindestens einer von Unregelmäßigkeit oder Verdickung in der Pleuralinie auf Basis eines Vergleichens (S244) der aggregierten Quantifizierung der Pleuralinie mit dem vorbestimmten Wert.

11. System (100) zur Verarbeitung eines Ultraschalleinzelbildes, umfassend:
eine Ultraschallsteuereinheit (150), die einen Speicher (151), der Anweisungen speichert, und einen Prozessor (152), der die Anweisungen ausführt, umfasst, wobei die Anweisungen, wenn sie vom Prozessor (152) ausgeführt werden, die Ultraschallsteuereinheit (150) veranlassen:
ein Ultraschalleinzelbild einer Lunge zu erhalten (S210) und eine Pleuralinie in der Lunge zu identifizieren (S220);
die in der Lunge identifizierte Pleuralinie zu quantifizieren (S230), um eine Quantifizierung der Pleuralinie zu erhalten;
die Quantifizierung der Pleuralinie mit einem vorbestimmten Wert zu vergleichen (S240); und
mindestens eine von Unregelmäßigkeit oder Verdickung in der Pleuralinie auf Basis des Vergleichs der Quantifizierung der Pleuralinie mit dem vorbestimmten Wert zu identifizieren (S250),
**dadurch gekennzeichnet, dass** die Anweisungen ferner die Ultraschallsteuereinheit (150) veranlassen, eine Angabe der mindestens einen von Unregelmäßigkeit oder Verdickung in der Pleuralinie auszugeben (S280),
wobei die Angabe als Daten, die dem Ultraschalleinzelbild überlagert sind, ausgegeben wird.

12. System (100) nach Anspruch 11, ferner umfassend:
eine Anzeige (180), die die Angabe der mindestens einen von Unregelmäßigkeit oder Verdickung in der Pleuralinie anzeigt.

13. System (100) nach Anspruch 11, wobei die Anweisungen, wenn sie vom Prozessor (152) ausgeführt werden, die Ultraschallsteuereinheit (150) ferner veranlassen:
ein trainiertes künstliches Intelligenzmodell auf die Quantifizierung der Pleuralinie anzuwenden, um die Quantifizierung der Pleuralinie mit dem vorbestimmten Wert zu vergleichen (S240) und mindestens eine von Unregelmäßigkeit oder Verdickung zu identifizieren (S250).

14. System (100) nach Anspruch 11, wobei die Anweisungen, wenn sie vom Prozessor (152) ausgeführt werden, die Ultraschallsteuereinheit (150) ferner veranlassen:
eine in der Pleuralinie identifizierte Unregelmäßigkeit zu quantifizieren, wobei die Unregelmäßigkeit mindestens ein Maß für Diskontinuität, Glätte oder Intensitätsvariation umfasst.

15. System (100) nach Anspruch 11, wobei die Anweisungen, wenn sie vom Prozessor (152) ausgeführt werden, die Ultraschallsteuereinheit (150) ferner veranlassen:
eine in der Pleuralinie identifizierte Verdickung zu quantifizieren, wobei die Verdickung mindestens ein Maß für eine Breite der Pleuralinie, Dicke einer Segmentierung der Pleuralinie oder Fläche der Segmentierung der Pleuralinie umfasst.

## Revendications

1. Support de stockage tangible non transitoire lisible par ordinateur (782) qui stocke un programme informatique (784), dans lequel le programme informatique (784), lorsqu'il est exécuté par un processeur (152, 710), amène un appareil informatique (110, 700) à effectuer les étapes d'un procédé comprenant :
l'obtention (S210) d'une trame échographique d'un poumon et l'identification (S220) d'une ligne pleurale dans le poumon ;
la quantification (S230) de la ligne pleurale identifiée dans le poumon pour obtenir une quantification de la ligne pleurale ;
la comparaison (S240) de la quantification de la ligne pleurale à une valeur prédéterminée qui caractérise une ligne pleurale normale et saine ; et
l'identification (S250) d'au moins une irrégularité ou un épaississement de la ligne pleurale en comparant la quantification de la ligne pleurale à la valeur prédéterminée,
**caractérisé en ce qu'**il comprend en outre l'émission (S280) d'une indication de l'au moins une irrégularité ou un épaississement de la ligne pleurale,
dans lequel l'indication est affichée sous forme de données superposées à la trame échographique sur un écran (180, 750).

2. Support de stockage tangible non transitoire lisible par ordinateur (782) selon la revendication 1, dans lequel le procédé comprend en outre l'étape de :
détermination (S260) d'un degré de l'au moins un élément parmi une irrégularité ou un épaississement de la ligne pleurale en fonction de la quantification de la ligne pleurale.

3. Support de stockage tangible non transitoire lisible par ordinateur (782) selon la revendication 2, dans lequel la détermination (S260) du degré de l'au moins un élément parmi une irrégularité ou un épaississement de la ligne pleurale comprend au moins un parmi :
- la détermination d'un niveau de gravité associé à la ligne pleurale ;
- la détermination si une ligne pleurale est normale ou anormale ;
- la détermination, sur la base de la quantification de la ligne pleurale, d'une évaluation de l'état général du poumon ; ou
- la détermination, sur la base de la quantification de la ligne pleurale, d'une probabilité d'une ou plusieurs affections pathologiques.

4. Support de stockage tangible non transitoire lisible par ordinateur (782) selon la revendication 2, dans lequel le programme informatique (784), lorsqu'il est exécuté par un processeur (152, 710), amène en outre l'appareil informatique (110, 700) à extraire des caractéristiques d'image montrant une irrégularité de la ligne pleurale, et
dans lequel la détermination (S260) du degré d'irrégularité est basée sur des mesures des caractéristiques de l'image.

5. Support de stockage tangible non transitoire lisible par ordinateur (782) selon la revendication 1, dans lequel le procédé comprend en outre l'étape de :
quantification d'une irrégularité identifiée dans la ligne pleurale, dans lequel l'irrégularité comprend au moins une mesure de discontinuité, de régularité ou de variation d'intensité.

6. Support de stockage tangible non transitoire lisible par ordinateur (782) selon la revendication 1, dans lequel le procédé comprend en outre l'étape de :
quantification de l'épaississement identifié dans la ligne pleurale, dans lequel l'épaississement comprend au moins une mesure de la largeur de la ligne pleurale, de l'épaisseur d'une segmentation de la ligne pleurale ou de la surface de la segmentation de la ligne pleurale.

7. Support de stockage tangible non transitoire lisible par ordinateur (782) selon la revendication 1, dans lequel le dispositif de quantification (S230) comprend en outre :
le calcul (S232) d'une pluralité de mesures de la ligne pleurale.

8. Support de stockage tangible non transitoire lisible par ordinateur (782) selon la revendication 7, dans lequel le dispositif de comparaison (S240) comprend en outre :
la comparaison (S242) de chacune des mesures de la ligne pleurale à une valeur prédéterminée correspondante.

9. Support de stockage tangible non transitoire lisible par ordinateur (782) selon la revendication 1, dans lequel le procédé comprend en outre l'étape de :
détection par imagerie échographique du poumon ; et
exécution automatique par l'appareil informatique (110, 700) du procédé basé sur la détection de l'imagerie échographique du poumon.

10. Support de stockage tangible non transitoire lisible par ordinateur (782) selon la revendication 1, dans lequel le procédé comprend en outre l'étape de :
agrégation (S234) de la quantification de la ligne pleurale identifiée dans le poumon à partir de la trame échographique avec une autre quantification de la ligne pleurale identifiée dans le poumon à partir d'une autre trame échographique pour obtenir une quantification agrégée de la ligne pleurale, et
identification (S250) d'au moins une irrégularité ou un épaississement de la ligne pleurale en comparant (S244) la quantification agrégée de la ligne pleurale à la valeur prédéterminée.

11. Système (100) de traitement d'une trame échographique, comprenant :
un dispositif de commande à ultrasons (150) comprenant une mémoire (151) qui stocke des instructions et un processeur (152) qui exécute ces instructions, dans lequel, lorsqu'elles sont exécutées par le processeur (152), les instructions amènent le dispositif de commande à ultrasons (150) à :
obtenir (S210) une trame échographique d'un poumon et identifier (S220) une ligne pleurale dans le poumon ;
quantifier (S230) la ligne pleurale identifiée dans le poumon pour obtenir une quantification de la ligne pleurale ;
comparer (S240) la quantification de la ligne pleurale à une valeur prédéterminée ; et
identifier (S250) au moins une irrégularité ou un épaississement de la ligne pleurale en comparant la quantification de la ligne pleurale à la valeur prédéterminée,
**caractérisé en ce que** les instructions amènent en outre le dispositif de commande à ultrasons (150) à émettre (S280) une indication d'au moins une irrégularité ou un épaississement de la ligne pleurale,
dans lequel l'indication est affichée sous forme de données superposées à la trame échographique.

12. Procédé (100) selon la revendication 11, comprenant en outre :
un écran (180) qui affiche l'indication de l'au moins une irrégularité ou un épaississement de la ligne pleurale.

13. Système (100) selon la revendication 11, dans lequel, lorsqu'elles sont exécutées par le processeur (152), les instructions amènent en outre dispositif de commande à ultrasons (150) à :
appliquer un modèle d'intelligence artificielle entraîné à la quantification de la ligne pleurale pour comparer (S240) la quantification de la ligne pleurale à la valeur prédéterminée et pour identifier (S250) au moins une irrégularité ou un épaississement.

14. Système (100) selon la revendication 11, dans lequel, lorsqu'elles sont exécutées par le processeur (152), les instructions amènent en outre dispositif de commande à ultrasons (150) à :
quantifier une irrégularité identifiée dans la ligne pleurale, dans lequel l'irrégularité comprend au moins une mesure de discontinuité, de régularité ou de variation d'intensité.

15. Système (100) selon la revendication 11, dans lequel, lorsqu'elles sont exécutées par le processeur (152), les instructions amènent en outre dispositif de commande à ultrasons (150) à :
quantifier l'épaississement identifié dans la ligne pleurale, dans lequel l'épaississement comprend au moins une mesure de la largeur de la ligne pleurale, de l'épaisseur d'une segmentation de la ligne pleurale ou de la surface de la segmentation de la ligne pleurale.
